# EUROPEAN PATENT APPLICATION

(11) **EP 4 563 569 A1**
(43) Date of publication of application: **04.06.2025**
(21) Application number: 22956282.2
(22) Date of filing: 02.11.2022
(51) Int. Cl.: C07C 233/83, C07C 237/46, C07C 233/76, C07C 231/02, A61L 24/00, A61L 24/04

(54) **AMIDE COMPOUND CONTAINING IODO ARYL OR IODO HETEROARYL, AND PREPARATION METHOD THEREFOR AND USE THEREOF**

(30) Priority: 25.08.2022 CN 202211028536; 25.08.2022 CN 202211027266; 25.08.2022 CN 202211027271
(71) Applicant: Cardiolink Science (Shenzhen) Medical Technology Development Co., Ltd., Shenzhen, Guangdong 518118 (CN)
(72) Inventor: SUN, Hongtao, Shenzhen, Guangdong 518118 (CN); LI, Xin, Shenzhen, Guangdong 518118 (CN); XIAO, Jinpeng, Shenzhen, Guangdong 518118 (CN); SUN, Peng, Shenzhen, Guangdong 518118 (CN); CHE, Haibo, Shenzhen, Guangdong 518118 (CN)
(74) Representative: TBK
(86) International application number: PCT/CN2022/129235
(87) International publication number: WO 2024/040745

(57) **Abstract**

The present application provides an amide compound containing iodo aryl or iodo heteroaryl, and a preparation method therefor and a use thereof. The amide compound containing iodo aryl or iodo heteroaryl can be used for preparing an X-ray embolism imaging material, so that the imaging modification efficiency is improved, and the iodine content of the X-ray embolism imaging material is increased.

## Description

### TECHNICAL FIELD

The present application belongs to the technical field of material synthesis, and relates to an amide compound having iodoaryl or iodoheteroaryl, a preparation method therefor and a use thereof.

### BACKGROUND

Due to the strong absorption of X-ray by iodine atoms, iodine-containing compounds, especially iodine-containing organic compounds, can be imageable in human body by X-ray detection. The currently commonly used clinical contrast medium molecules, such as diatrizoate, iohexol, iodixanol, iopamidol, iotrolan, etc., with both high water solubility and high iodine content, are used for angiography of blood vessels and organs under X-ray detection such as CT in clinical interventional operations, which greatly facilitate clinical surgical operations.

Lipiodol, a liquid embolic material commonly used in clinical TACE (Transhepatic Arterial Chem Otherapy And Embolization) procedures, is mainly composed of poppy oil substituted by iodine. As an embolic material, the lipiodol is visible in the human body under X-ray detection, which facilitates clinicians to directly evaluate the embolization effect. Patent CN102781974B discloses the process that 2,3,5-triiodobenzyl bromide and polyethylene glycol (PVA) form ether bonds under the action of strong alkali, and then emulsion precipitation is performed to obtain iodine-containing nanoparticles, which can be used as an embolic material for X-ray imaging. Patent CN104717983B discloses a poly-2-hydroxypropionic acid solution modified with triiodophenol and iohexol, which is used to generate imaging embolic precipitation in situ after injected into blood. Patent CN113651906A discloses the process that an iodophenyl ether compound is grafted with PVA to obtain a liquid embolic material similar to ONYX glue. This embolic material generates solid precipitate in situ after injected into the blood vessel, forming an embolus that is visible under X-ray.

In addition, the gel microspheres modified with iodine-containing organic compound molecules as solid imaging embolic agents under X-ray have also been rapidly developed in recent years. Patents CN105517582B and CN112334497A of BTG, UK disclose the process that an iodoaryl aldehyde or acetal compound is used to modify a polyvinyl alcohol microsphere under an acidic condition to obtain an imaging microsphere for embolization. An alkyl or alkoxy chain is used to connect iodoaryl to aldehyde in the modified molecules synthesized in the patent application, resulting in a limited range of molecules in this class, which require multi-step synthesis and are difficult to synthesize. Patent CN111821503A discloses the process that an iodoaryl chloride or sulfonyl chloride compound is used to modify a PVA microsphere to obtain an embolic microsphere with 31.9-52.6% iodine content. The use of aryl chloride or sulfonyl chloride to modify the microsphere is harsh in conditions and unfavorable for process scale-up. Besides, patent CN105517580A discloses the process that imidazole carbonate and PVA microspheres are reacted first and then reacted with an iodine-containing alcohol or amine to achieve iodine modification of gel microspheres. This method has mild reaction conditions, but requires a two-step reaction, which is a cumbersome process; moreover, the carbonate bond is used to connect the imaging molecules to microspheres, which is less stable.

Therefore, it has significant importance to further develop X-ray imaging materials in this field.

### SUMMARY

The present application provides an amide compound having iodoaryl or iodoheteroaryl, a preparation method therefor and a use thereof. The amide compound having iodoaryl or iodoheteroaryl of the present application can be used to prepare an X-ray imaging embolic material. The compound of the present application is simple to synthesize, contains an amide structure in the molecule, and has a stable structure and good hydrophilicity. The microsphere after imaging modification has better hydrophilicity, elasticity and stability.

In a first aspect, the present application provides an amide compound having iodoaryl or iodoheteroaryl, and the compound has a structure shown in formula I: 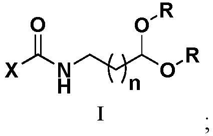 wherein, X is C₅₋₁₂ aryl substituted by at least one iodine atom, C₅₋₁₂ heteroaryl substituted by at least one iodine atom, C₅₋₁₂ aryloxy-C₁₋₄ alkylene substituted by at least one iodine atom, or C₅₋₁₂ aryl substituted by at least one iodine atom; R is C₁₋₆ alkyl; and n is an integer greater than or equal to 0.

In the present application, in the case where a group is defined for the amide compound having iodoaryl or iodoheteroaryl, the number of carbon atom in the group is limited. In the numerical range of the limited number of carbon atom, any integer of the given numerical range can represent the number of carbon atom in the group. For example, C₁₋₆ alkyl means that the number of carbon atom in the alkyl group can be 1, 2, 3, 4, 5 or 6.

Preferably, for the compound of formula I, n is equal to 0, 1, 2 or 3.

Preferably, X is C₅₋₇ aryl substituted by at least one iodine atom, and further preferably, X is phenyl substituted by at least one iodine atom.

Preferably, X is selected from any one of the following groups: 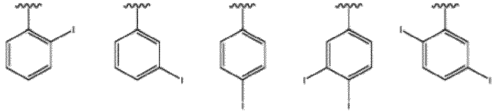 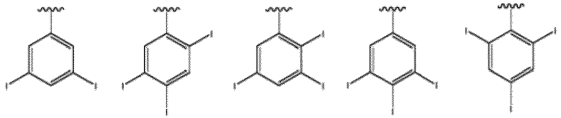 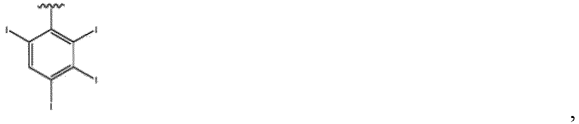 wherein the wavy line represents a linkage site of a group;

Preferably, R is methyl, ethyl, n-propyl, isopropyl, n-butyl or n-hexyl.

Preferably, the amide compound having iodoaryl or iodoheteroaryl is any one of the following compounds:

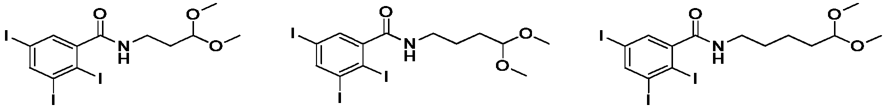

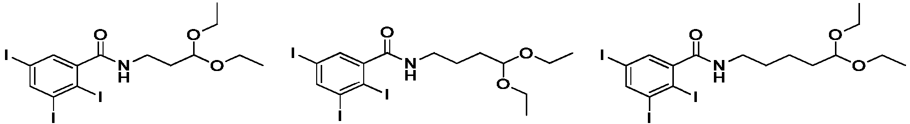

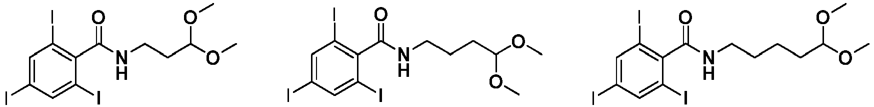

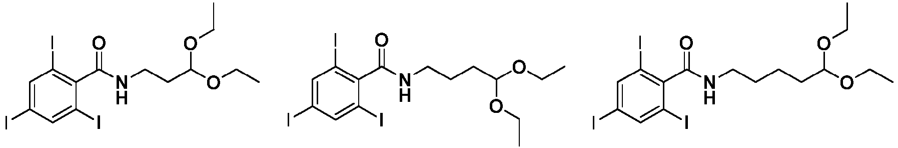

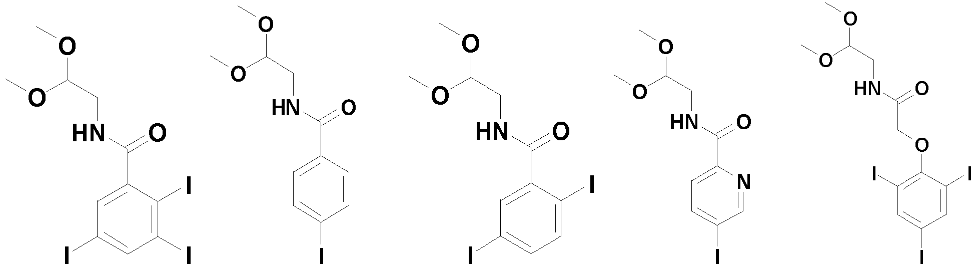

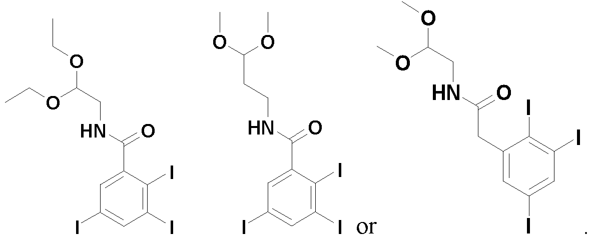

In a second aspect, the present application provides a method of preparing the amide compound having iodoaryl or iodoheteroaryl as above, and the preparation method includes the following steps:
carrying out a condensation reaction on a compound of formula V and a compound of formula VI to obtain the amide compound having iodoaryl or iodoheteroaryl shown in formula I, the reaction formula of which is as follows: 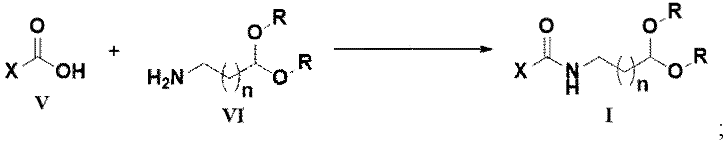 wherein X, R and n are defined as above.

Preferably, the compound of formula VI and the compound of formula V have a molar ratio of 1:1-10:1, such as 1:1, 2:1, 3:1, 4:1, 5:1, 6:1, 7:1, 8:1, 9:1 or 10:1.

Preferably, the condensation reaction is carried out in the presence of a condensation reagent.

Preferably, the condensation reagent is a combination of 1-(3-dimethylaminopropyl)-3-ethylcarbodiimide hydrochloride and 1-hydroxybenzotriazole, or 2-(7-azobenzotriazol-1-yl)-N,N,N',N'-tetramethyluronium hexafluorophosphate.

Preferably, the condensation reagent and the compound of formula VI have a molar ratio of 1:1-6:1, such as 1:1, 1.5:1, 1.8:1, 2:1, 2.5:1, 2.8:1, 3:1, 3.5:1, 4:1, 4.5:1, 5:1 or 6:1.

In the case where the condensation reagent is a combination of 1-(3-dimethylaminopropyl)-3-ethylcarbodiimide hydrochloride and 1-hydroxybenzotriazole, 1-(3-dimethylaminopropyl)-3-ethylcarbodiimide hydrochloride and 1-hydroxybenzotriazole have a molar ratio of 1:1.

Preferably, the condensation reaction is carried out in the presence of a basic substance, wherein the basic substance is triethylamine, N,N-diisopropylethylamine, triethylenediamine (DABCO) or N-methylmorpholine.

Preferably, a solvent of the condensation reaction is any one or a combination of at least two of N,N-dimethylformamide, N,N-dimethylacetamide, dichloromethane or tetrahydrofuran.

Preferably, the condensation reaction is carried out at room temperature for 2-48 h, such as 2 h, 5 h, 8 h, 10 h, 12 h, 15 h, 18 h, 20 h, 24 h, 28 h, 32 h, 36 h, 38 h, 40 h, 44 h or 48 h.

Preferably, the condensation reaction is carried out under the protection of a protective gas, wherein the protective gas is preferably nitrogen.

In a third aspect, the present application provides an X-ray imaging material, and the X-ray imaging material includes the amide compound having iodoaryl or iodoheteroaryl as above.

Preferably, the X-ray imaging material is an X-ray imaging embolic microsphere.

Preferably, the X-ray imaging material is a radiopaque embolic microsphere, wherein the radiopaque embolic microsphere includes a polyvinyl alcohol microsphere and an imaging molecule which are connected in a manner of 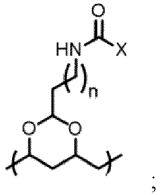 wherein X is C₅₋₁₂ aryl substituted by at least one iodine atom or C₅₋₁₂ heteroaryl substituted by at least one iodine atom; n is 0, 1, 2 or 3.

In the present application, the radiopaque embolic microsphere is obtained by attaching an iodine-containing contrast medium to a polyvinyl alcohol microsphere as the main body.

When n equals to 0, the microsphere structure is unstable and easily produces degradation impurities during the sterilization process; and the microsphere with this structure remains strong and difficult to compress. By appropriately extending the chain length, the stability of microsphere is improved. In addition, the alkyl chain length increased also means that the flexibility of the side chain is increased, which allows the microsphere to be less strong and more elastic, and thus the embolization performance can be better. Furthermore, this structure will also give the embolic microsphere better drug loading performance.

The amide compound having iodoaryl or iodoheteroaryl, which has aldehyde, acetal or semiacetal with an extended group, is used as the imaging molecule in the embolic microsphere of the present application. The introduction of the amide structure increases the hydrophilicity of the imaging microsphere, the extended structure increases the flexibility of the microsphere, the microsphere is easier to achieve the imaging modification, the imaging embolic material is allowed to have better hydrophilicity and flexibility, and the imaging embolic microsphere is significantly improved in drug loading capacity and drug delivery rate.

Polyvinyl alcohol of the polyvinyl alcohol microsphere in the present application has a molecular mass of 2000-10000000, such as 2000, 3000, 5000, 10000, 30000, 50000, 80000, 300000, 500000, 800000 or 1000000.

Preferably, the radiopaque embolic microsphere has the following structure: 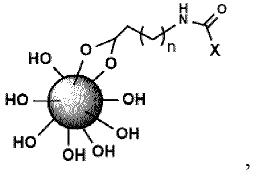 wherein 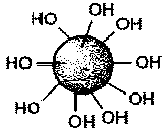 represents the polyvinyl alcohol microsphere, and the structural formula connected to the polyvinyl alcohol microsphere is derived from the imaging molecule, and X and n are defined as above.

Preferably, the polyvinyl alcohol microsphere has an average diameter of 10-2000 µm, such as 10 µm, 50 µm, 80 µm, 100 µm, 200 µm, 500 µm, 800 µm, 1000 µm, 1200 µm, 1500 µm or 2000 µm, more preferably 30-1200 µm, and most preferably 40-500 µm.

In a fourth aspect, the present application also provides a preparation method of the X-ray imaging material as above, and the preparation method includes the following steps: adding a polymer microsphere into a solvent for swelling, then adding the imaging molecule and methanesulfonic acid, and carrying out a reaction to obtain the X-ray imaging material.

Preferably, the solvent is dimethyl sulfoxide (DMSO).

Preferably, in the reaction system, the polymer microsphere has a mass percent of 1%-50%, such as 1%, 3%, 5%, 8%, 10%, 15%, 20%, 25%, 30%, 35%, 40%, 45% or 50%.

Preferably, in the reaction system, the imaging molecule has a molar concentration of 0.01-5 mol/L, such as 0.03 mol/L, 0.1 mol/L, 0.5 mol/L, 0.8 mol/L, 1 mol/L, 1.5 mol/L, 2 mol/L, 2.5 mol/L, 3 mol/L, 3.5 mol/L, 4 mol/L, 4.5 mol/L or 5 mol/L.

Preferably, in the reaction system, the methanesulfonic acid has a molar concentration of 0.05-5 mol/L, such as 0.05 mol/L, 0.1 mol/L, 0.5 mol/L, 0.8 mol/L, 1 mol/L, 1.5 mol/L, 2 mol/L, 2.5 mol/L, 3 mol/L, 3.5 mol/L, 4 mol/L, 4.5 mol/L or 5 mol/L.

Preferably, the reaction is carried out at 50-90°C for 24-48 h.

Preferably, after the reaction, the product is washed with DMSO and a sodium bicarbonate solution in sequence.

In a fifth aspect, the present application provides a composition, and the composition includes a solvent and the X-ray imaging material as above.

In some embodiments, the solvent is a physiological saline solution.

The imaging microsphere modified with the amide compound having iodoaryl or iodoheteroaryl of the present application has better hydrophilicity and operability.

Compared with the prior art, the present application has the beneficial effects below.

In the compound of the present application, an amide bond is used to connect the iodoaryl or heteroaryl structure to the aldehyde or acetal structure of a specific length. By introducing the hydrophilic amide structure, the imaging embolic material is easier to achieve imaging modification when the compound is used to prepare the imaging embolic material. The prepared imaging embolic material has better hydrophilicity, better elasticity and better stability, and the imaging modification efficiency is improved, resulting in a higher iodine content of the X-ray imaging embolic material.

The amide compound having iodoaryl or iodoheteroaryl shown in formula I of the present application is prepared by condensation reaction in the present application. The reaction can be carried out at room temperature, the reaction conditions are mild, the reaction is simple and easy to operate, the product can be prepared by one-step reaction, the cost is low, and the yield is up to 70% or more.

The polyvinyl alcohol microsphere and the imaging molecule are connected together through an acetal structure in the embolic microsphere of the present application. By introducing a hydrophilic amide structure, the imaging embolic material has better hydrophilicity, and at the same time the binding effect of the imaging microsphere with the drug is enhanced. By increasing the length of the carbon chain of the imaging molecule, the flexibility of the imaging molecule is increased, the microsphere is easier to achieve the imaging modification, the stability of the intermediate aldehyde generated in the imaging modification reaction is greatly enhanced, the imaging modification efficiency is higher, the iodine content of the obtained imaging microsphere is higher and more uniform, and the drug loading capacity and drug delivery rate of the imaging embolic microsphere can be improved.

### BRIEF DESCRIPTION OF DRAWINGS

FIG. 1 shows a ¹H-NMR spectrum of Compound I-1.
FIG. 2 shows a LCMS spectrum of Compound I-1.
FIG. 3 shows a HPLC spectrum of Compound I-1.
FIG. 4 shows a ¹H-NMR spectrum of Compound 1-2'.
FIG. 5 shows a LCMS spectrum of Compound I-2'.
FIG. 6 shows a HPLC spectrum of Compound I-2'.
FIG. 7-a shows an optical image of imaging microspheres in Application Example 1.

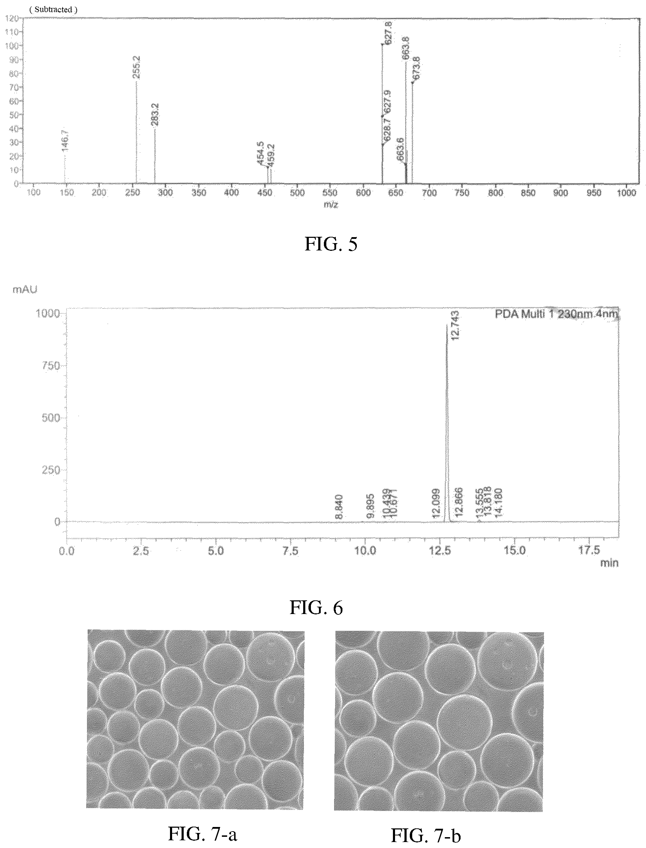

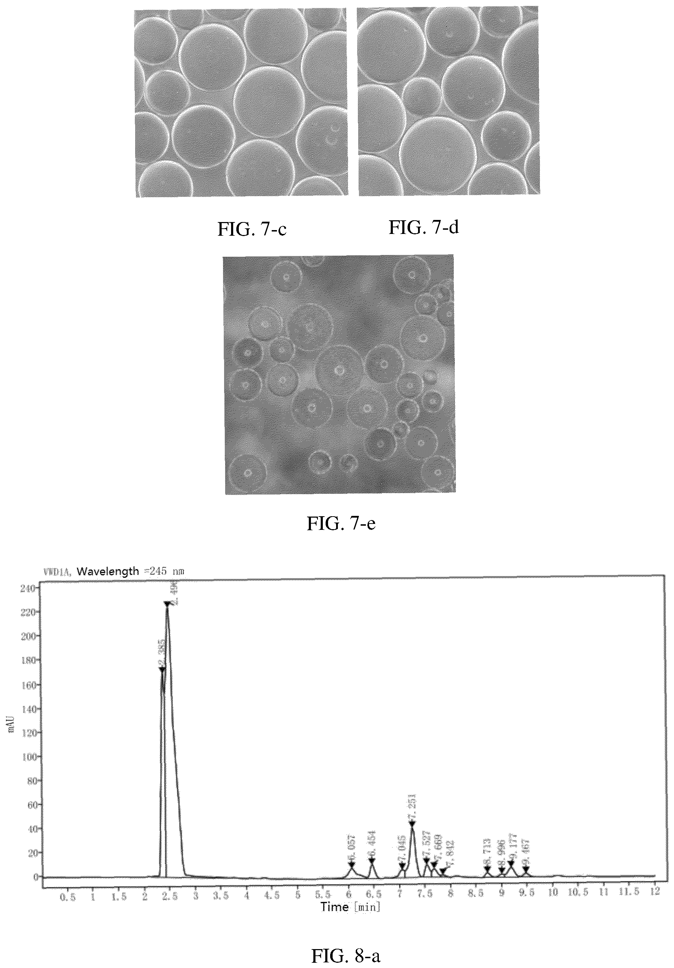

FIG. 7-b shows an optical image of imaging microspheres in Application Example 2.
FIG. 7-c shows an optical image of imaging microspheres in Application Example 3.
FIG. 7-d shows an optical image of imaging microspheres in Application Example 4.
FIG. 7-e shows an optical image of imaging microspheres in Application Example 5.

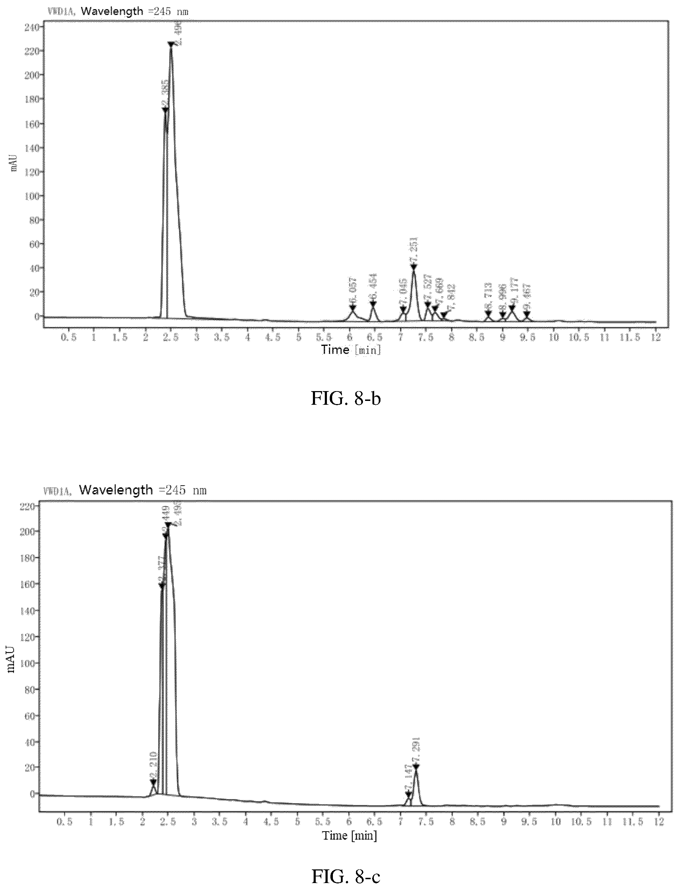

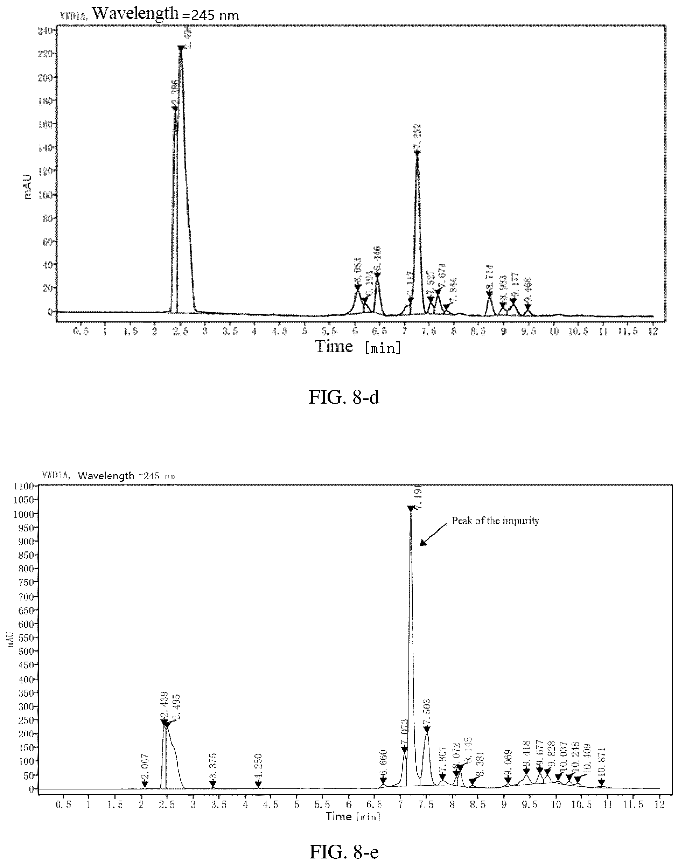

FIG. 8-a shows a HPLC spectrum of a reaction solution in Application Example 1.
FIG. 8-b shows a HPLC spectrum of a reaction solution in Application Example 2.
FIG. 8-c shows a HPLC spectrum of a reaction solution in Application Example 3.
FIG. 8-d shows a HPLC spectrum of a reaction solution in Application Example 4.
FIG. 8-e shows a HPLC spectrum of a reaction solution in Application Example 5.

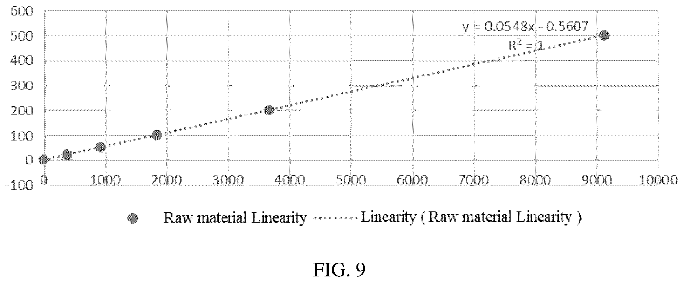

FIG. 9 shows a standard curve of Compound IV, an impurity.

### DETAILED DESCRIPTION

The technical solutions of the present application are further described below through embodiments. It should be apparent to those skilled in the art that the embodiments are only used for a better understanding of the present application and should not be construed as a specific limitation of the present application.

Compounds obtained in examples of the present application were characterized by HPLC (High Performance Liquid Chromatography), LCMS (Liquid Chromatography Mass Spectrometry) and ¹H-NMR (Hydrogen Nuclear Magnetic Resonance Spectroscopy), and the methods are described below.

HPLC: The test instrument was Agilent 1260 high performance liquid chromatograph; the column was an octadecyl bonded silica gel column; the mobile phase was 0.1% phosphoric acid aqueous solution and methanol; the detector was a VWD detector; about 0.1 mg of the sample was added into a sample bottle, dissolved with DMSO and then directly injected into the sample injector for detection; the product purity was determined according to an integrated area.

LCMS: The test instrument was Agilent 1260-6125 high performance liquid chromatograph -mass spectrometer; the column was an octadecyl bonded silica gel column; the mobile phase was 0.1% formic acid aqueous solution and methanol; the detector was a detector of the mass spectrometer; about 0.1 mg of the sample was added into a sample bottle, dissolved with DMSO and then directly injected into the sample injector for detection, and the main ion peaks were identified.

¹H-NMR: The test instrument was Bruker 400 M NMR spectrometer; the probe was a room temperature probe for ¹H-NMR spectrum; 20-30 mg of the sample was added into a NMR tube, dissolved with 0.3-0.5 mL of deuterated DMSO and then subjected to detection.

### Preparation Example 1

### Preparation of Compound I-1

### Method A:

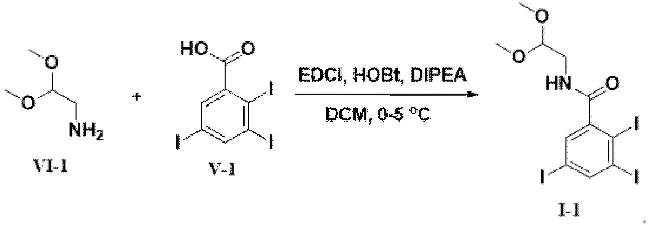

Compound V-1 (90 g 1.0 eq) and DCM (7 L) were added into a reaction flask in sequence, and the system was stirred, added with Compound VI-1 (1.5 eq), cooled to 0-5 °C, subjected to nitrogen protection, and then added with DIPEA (1.5 eq), HOBt (1.5 eq) and EDCI (1.5 eq). After the addition, the system was transferred to room temperature and stirred for 16 h, and monitored by TLC. When the raw materials were completely reacted, water (10 L) and DCM (3 L) were added. The system was stirred for 5 min, allowed to stand and separated into phases. The organic phase was concentrate to dryness, added with EA (4 L) and PE (3 L), and pulped at room temperature for 1 h. The mixture was filtered and dried by rotary evaporation to obtain the crude compound.

The I-1 crude product can be further purified. The obtained compound and THF (3 L) were added into a reaction flask, stirred for 5 min, and filtered. The obtained filtrate was added with THF (1 L) and activated carbon (0.2 wt%), heated to 60°C, stirred for 1 h, filtered with diatomite, and washed by dribbling a small amount of THF. The filtrate was concentrated to precipitate a small amount of solid, added with n-heptane (5 L), stirred at room temperature for 10 min, filtered, and washed by dribbling n-heptane. The obtained solid was dried by rotary evaporation to obtain 85.67 g of Compound I-1 with a yield of 81% and a purity of 98.98%.

### Method B:

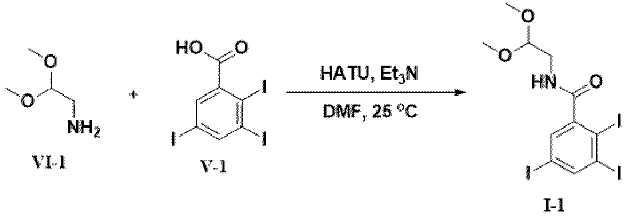

Under nitrogen protection, Compound VI-1 (1.2 eq.), Compound V-1 (1.0 eq., 50 g), HATU (1.5 eq.) and anhydrous DMF (5000 mL) were added into a single-necked reaction flask in sequence, stirred until dissolved, then added with triethylamine (3.0 eq.) slowly, and kept at room temperature for reaction. Under TLC monitoring, when the raw materials were completely reacted, 5000 mL of water was added into the reaction solution to quench the reaction, and then 5000 mL of ethyl acetate (EA) was added for extraction and layer separation. The organic phase was taken, washed three times with water (5000 mL × 3), dried with anhydrous sodium sulfate, concentrated to a certain extent (about 300 mL of EA was remained) to precipitate a large amount of solid, cooled to -5-0 °C, stirred to precipitate a solid, and then subjected to suction filtration. The filter cake was washed by dribbling a small amount of cold EA, and dried under vacuum at 45 °C for 2 h to obtain the target product I-1 (44 g, 75% yield).

Compound I-1 is a solid, which varies in color from white to light pink and has a melting point of 180-182 °C.

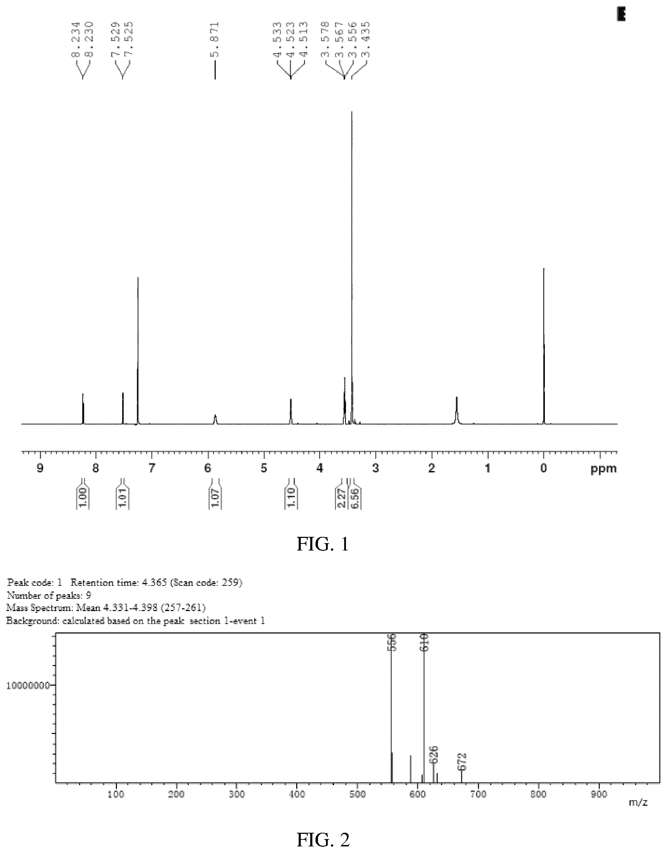

The ¹H-NMR spectrum of Compound I-1 is shown in FIG. 1. ¹H NMR (400 MHz, DMSO-*d₆*) *δ* 8.58 (t, *J =* 5.9 Hz, 1 H), 8.25 (d, *J =* 1.9 Hz, 1 H), 7.46 (d, *J =* 1.9 Hz, 1 H), 4.48 (t, *J* = 5.5 Hz, 1 H), 3.29 (s, 6 H), 3.28-3.25 (m, 2 H).

The LCMS spectrum of Compound I-1 is shown in FIG. 2, MS (EI): m/z 588 (M + H⁺), 610 (M + Na⁺).

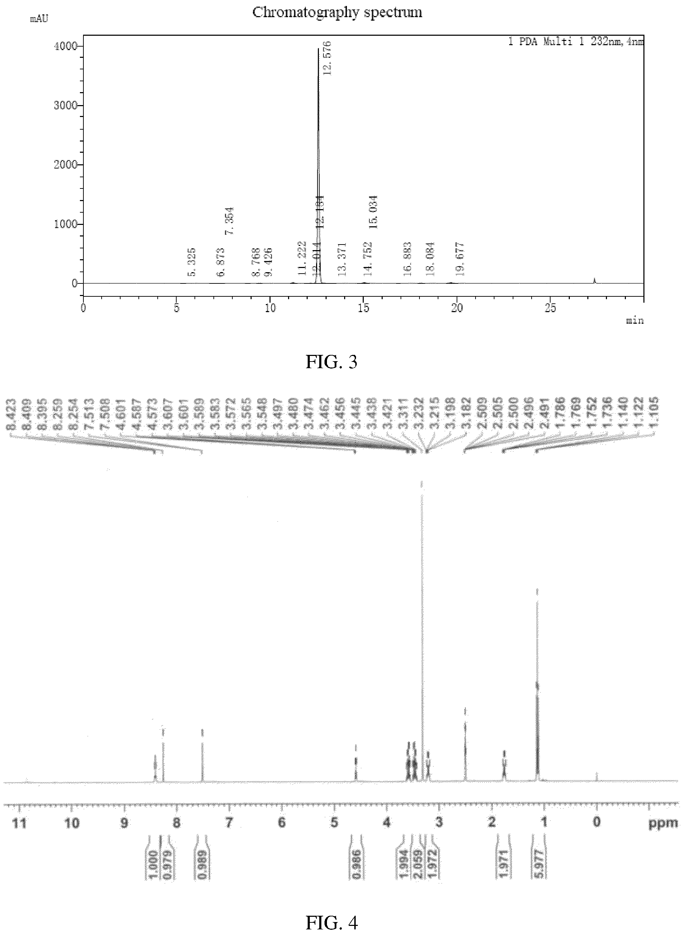

FIG. 3 shows the HPLC spectrum of Compound I-1.

It can be found from FIGs. 1-3 that the synthesized compound is a single target compound, Compound I-1, with a purity of > 98%.

### Preparation Example 2

### Preparation of Compound I-1'

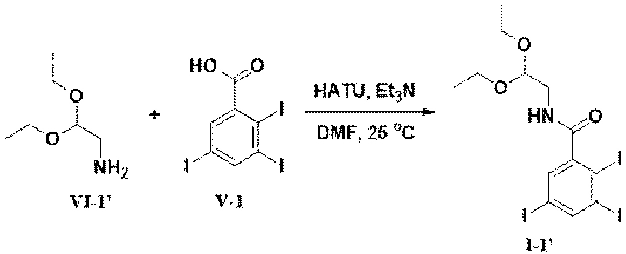

Compound I-1' was prepared by Method B. The specific reaction steps were described below.

Under nitrogen protection, Compound VI-1' (1.2 eq.), Compound V-1 (1.0 eq., 50 g), HATU (1.5 eq.) and anhydrous DMF (5000 mL) were added into a single-necked reaction flask in sequence, stirred until dissolved, then added with triethylamine (3.0 eq.) slowly, and kept at room temperature for reaction. Under TLC monitoring, when the raw materials were completely reacted, 5000 mL of water was added into the reaction solution to quench the reaction, and then 5000 mL of ethyl acetate (EA) was added for extraction and layer separation. The organic phase was taken, washed three times with water (5000 mL × 3), dried with anhydrous sodium sulfate, concentrated to a certain extent (about 300 mL of EA was remained) to precipitate a large amount of solid, cooled to -5-0 °C, stirred to precipitate a solid, and then subjected to suction filtration. The filter cake was washed by dribbling a small amount of cold EA, and dried under vacuum at 45 °C for 2 h to obtain 54.8 g of the target product (89% yield). Compound I-1' is a white solid (98% purity) with a melting point of 150-152 °C. ¹H NMR (400 MHz, DMSO-*d₆*) *δ* 8.58 (t, *J =* 5.9 Hz, 1 H), 8.25 (d, *J =* 1.9 Hz, 1 H), 7.46 (d, *J =* 1.9 Hz, 1 H), 4.48 (t, *J =* 5.5 Hz, 1 H), 3.25 (q, *J =* 7.6 Hz, 4 H), 3.28-3.25 (m, 2 H), 1.25 (t, *J =* 7.6 Hz, 6 H); MS (EI): m/z 616 (M + H⁺).

### Preparation Example 3

### Preparation of Compound I-2

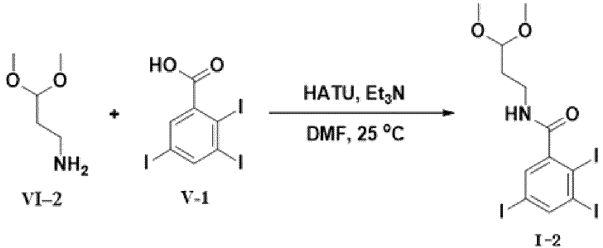

Under nitrogen protection, Compound VI-2 (1.2 eq.), Compound V-1 (1.0 eq., 50 g), HATU (1.5 eq.) and anhydrous DMF (5000 mL) were added into a single-necked reaction flask in sequence, stirred until dissolved, then added with triethylamine (3.0 eq.) slowly, and kept at room temperature for reaction. Under TLC monitoring, when the raw materials were completely reacted, 5000 mL of water was added into the reaction solution to quench the reaction, and then 5000 mL of ethyl acetate (EA) was added for extraction and layer separation. The organic phase was taken, washed three times with water (5000 mL × 3), dried with anhydrous sodium sulfate, concentrated to a certain extent (about 300 mL of EA was remained) to precipitate a large amount of solid, cooled to -5-0 °C, stirred to precipitate a solid, and then subjected to suction filtration. The filter cake was washed by dribbling a small amount of cold EA, and dried under vacuum at 45 °C for 2 h to obtain 48.9 g of the target product I-2 (80% yield, > 98% purity), which was a white solid with a melting point of 163-165 °C. ¹H NMR (400 MHz, DMSO-*d₆*) *δ* 8.59 (t, *J =* 5.9 Hz, 1 H), 8.25 (d, *J =* 1.9 Hz, 1 H), 7.46 (d, *J =* 1.9 Hz, 1 H), 4.43 (t, *J =* 5.5 Hz, 1 H), 3.29 (s, 6 H), 3.28-3.25 (m, 2 H), 1.92-1.89 (m, 2 H); MS (EI): m/z 602 (M + H⁺).

### Preparation Example 4

### Preparation of Compound I-2'

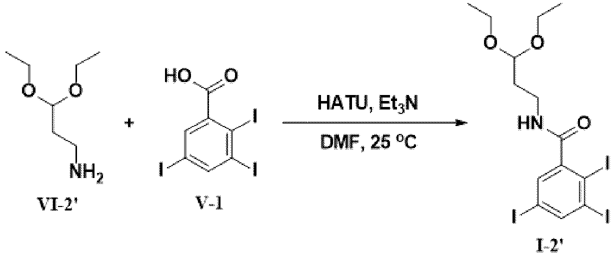

Under nitrogen protection, Compound VI-2' (1.2 eq.), Compound V-1 (1.0 eq., 50 g), HATU (1.5 eq.) and anhydrous DMF (5000 mL) were added into a single-necked reaction flask in sequence, stirred until dissolved, then added with triethylamine (3.0 eq.) slowly, and kept at room temperature for reaction. Under TLC monitoring, when the raw materials were completely reacted, 5000 mL of water was added into the reaction solution to quench the reaction, and then 5000 mL of ethyl acetate (EA) was added for extraction and layer separation. The organic phase was taken, washed three times with water (5000 mL × 3), dried with anhydrous sodium sulfate, concentrated to a certain extent (about 300 mL of EA was remained) to precipitate a large amount of solid, cooled to -5-0 °C, stirred to precipitate a solid, and then subjected to suction filtration. The filter cake was washed by dribbling a small amount of cold EA, and dried under vacuum at 45 °C for 2 h to obtain the target product I-2' (44 g, 70% yield, > 98% purity), which was a white solid with a melting point of 157-160 °C.

FIG. 4 shows the ¹H-NMR spectrum of Compound I-2': ¹H NMR (400 MHz, DMSO-*d₆*) δ 8.41 (t, *J =* 5.6 Hz, 1 H), 8.26 (d, *J =* 1.8 Hz, 1 H), 7.51 (d, *J =* 1.8 Hz, 1 H), 4.59 (t, *J =* 5.6 Hz, 1 H), 3.62-3.54 (m, 2 H), 3.50-3.42 (m, 2 H), 3.23-3.18 (m, 2 H), 1.78-1.73 (m, 2 H), 1.12 (t, *J =* 7.0 Hz, 6 H).

FIG. 5 shows the LCMS spectrum of Compound I-2', MS (EI): m/z 630 (M + H⁺).

FIG. 6 shows the HPLC spectrum of Compound I-2'.

It can be found from FIGs. 4-6 that the synthesized compound is a single target compound, Compound I-2', with a purity of >98%.

### Preparation Example 5

### Preparation of Compound I-3

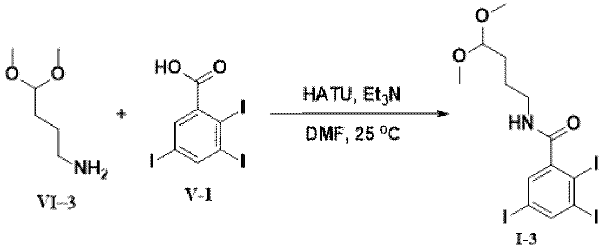

Under nitrogen protection, Compound VI-3 (2.0 eq.), Compound V-1 (1.0 eq., 50 g), HATU (1.5 eq.) and anhydrous DMF (5000 mL) were added into a single-necked reaction flask in sequence, stirred until dissolved, then added with triethylamine (3.0 eq.) slowly, and kept at room temperature for reaction. Under TLC monitoring, when the raw materials were completely reacted, 5000 mL of water was added into the reaction solution to quench the reaction, and then 5000 mL of ethyl acetate (EA) was added for extraction and layer separation. The organic phase was taken, washed three times with water (5000 mL × 3), dried with anhydrous sodium sulfate, concentrated to a certain extent (about 300 mL of EA was remained) to precipitate a large amount of solid, cooled to -5-0 °C, stirred to precipitate a solid, and then subjected to suction filtration. The filter cake was washed by dribbling a small amount of cold EA, and dried under vacuum at 45 °C for 2 h to obtain the target product I-3 (51.2 g, 83.7% yield, > 99% purity), which was a white solid with a melting point of 153-157 °C. ¹H NMR (400 MHz, DMSO-*d₆*) *δ* 8.41 (t, *J* = 5.6 Hz, 1 H), 8.26 (d, *J* = 1.8 Hz, 1 H), 7.51 (d, *J =* 1.8 Hz, 1 H), 4.57 (t, *J =* 5.6 Hz, 1 H), 3.28 (s, 6 H), 3.21-3.17 (m, 2 H), 1.81-1.77 (m, 2 H), 1.75-1.72 (m, 2 H); MS (EI): m/z 616 (M + H⁺).

### Preparation Example 6

### Preparation of Compound I-4

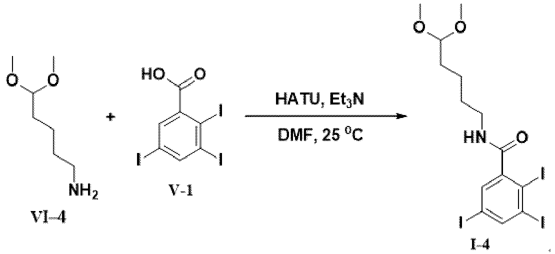

Under nitrogen protection, Compound VI-4 (1.5 eq.), Compound V-1 (1.0 eq., 50 g), HATU (1.5 eq.) and anhydrous DMF (5000 mL) were added into a single-necked reaction flask in sequence, stirred until dissolved, then added with triethylamine (3.0 eq.) slowly, and kept at room temperature for reaction. Under TLC monitoring, when the raw materials were completely reacted, 5000 mL of water was added into the reaction solution to quench the reaction, and then 5000 mL of ethyl acetate (EA) was added for extraction and layer separation. The organic phase was taken, washed three times with water (5000 mL × 3), dried with anhydrous sodium sulfate, concentrated to a certain extent (about 300 mL of EA was remained) to precipitate a large amount of solid, cooled to -5-0 °C, stirred to precipitate a solid, and then subjected to suction filtration. The filter cake was washed by dribbling a small amount of cold EA, and dried under vacuum at 45 °C for 2 h to obtain the target product I-4 (44.7 g, 71% yield, > 97% purity), which was a white solid with a melting point of 154-156 °C. ¹H NMR (400 MHz, DMSO-*d₆*) *δ* 8.41 (t, *J* = 5.6 Hz, 1 H), 8.26 (d, *J* = 1.8 Hz, 1 H), 7.51 (d, *J =* 1.8 Hz, 1 H), 4.57 (t, *J =* 5.6 Hz, 1 H), 3.28 (s, 6 H), 3.20-3.16 (m, 2 H), 1.81-1.77 (m, 2 H), 1.77-1.70 (m, 4 H); MS (EI): m/z 630 (M + H⁺).

### Preparation Example 7

### Preparation of Compound I-5

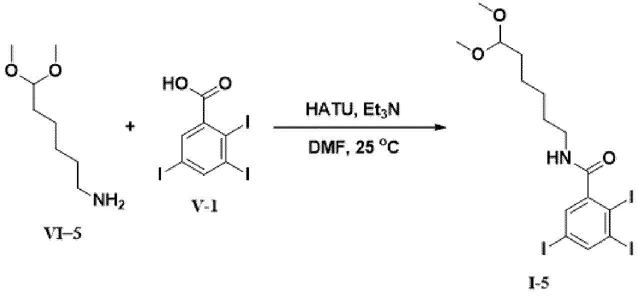

Under nitrogen protection, Compound VI-5 (10.0 eq.), Compound V-1 (1.0 eq., 50 g), HATU (1.5 eq.) and anhydrous DMF (5000 mL) were added into a single-necked reaction flask in sequence, stirred until dissolved, then added with triethylamine (3.0 eq.) slowly, and kept at room temperature for reaction. Under TLC monitoring, when the raw materials were completely reacted, 5000 mL of water was added into the reaction solution to quench the reaction, and then 5000 mL of ethyl acetate (EA) was added for extraction and layer separation. The organic phase was taken, washed three times with water (5000 mL × 3), dried with anhydrous sodium sulfate, concentrated to a certain extent (about 300 mL of EA was remained) to precipitate a large amount of solid, cooled to -5-0 °C, stirred to precipitate a solid, and then subjected to suction filtration. The filter cake was washed by dribbling a small amount of cold EA, and dried under vacuum at 45 °C for 2 h to obtain the target product I-5 (40.5 g, 63% yield, > 95% purity), which was a white solid with a melting point of 145-147 °C. ¹H NMR (400 MHz, DMSO-*d₆*) *δ* 8.42 (t, *J =* 5.6 Hz, 1 H), 8.27 (d, *J =* 1.8 Hz, 1 H), 7.52 (d, *J =* 1.8 Hz, 1 H), 4.58 (t, *J =* 5.6 Hz, 1 H), 3.29 (s, 6 H), 3.20-3.16 (m, 2 H), 1.81-1.77 (m, 2 H), 1.77-1.75 (m, 2 H), 1.74-1.70 (m, 4 H); MS (EI): m/z 630 (M + H⁺).

### Preparation Example 8

### Preparation of Compounds I-6

Compound I-6 was prepared by Method A. The specific reaction steps were described below.

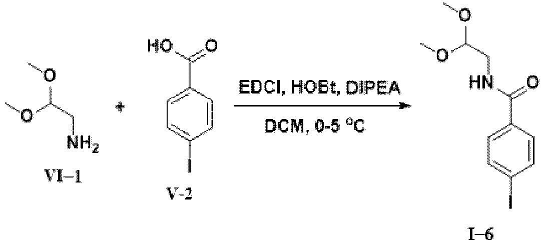

Compound V-2 (50 g 1.0 eq) and DCM (7 L) were added into a reaction flask in sequence, and the system was stirred, added with Compound VI-1 (1.5 eq), cooled to 0-5 °C, subjected to argon protection, and then added with DIPEA (1.5 eq), HOBt (1.5 eq) and EDCI (1.5 eq). After the addition, the system was transferred to room temperature and stirred for 16 h, and monitored by TLC. When the raw materials were completely reacted, water (10 L) and DCM (3 L) were added. The system was stirred for 5 min, allowed to stand and separated into phases. The organic phase was concentrate to dryness, added with EA (4 L) and PE (3 L), and pulped at room temperature for 1 h. The mixture was filtered and dried by rotary evaporation to obtain the crude compound.

The I-6 crude product can be further purified. The obtained compound and THF (3 L) were added into a reaction flask, stirred for 5 min, and filtered. The obtained filtrate was added with THF (1 L) and activated carbon (0.2 wt%), heated to 60 °C, stirred for 1 h, filtered with diatomite, and washed by dribbling a small amount of THF. The filtrate was concentrated to precipitate a small amount of solid, added with n-heptane (5 L), stirred at room temperature for 10 min, filtered, and washed by dribbling n-heptane. The obtained solid was dried by rotary evaporation to obtain 56.7 g of Compound I-6 with a yield of 84% and a purity of > 98%.

Compound I-6 was prepared by Method B. The specific reaction steps were described below.

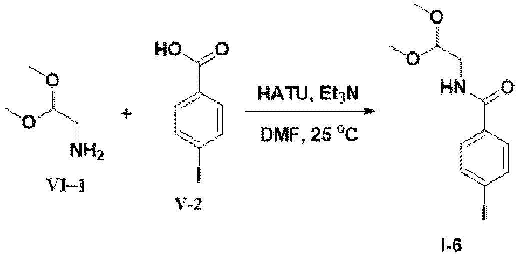

Under nitrogen protection, Compound VI-1 (1.2 eq.), Compound V-2 (1.0 eq., 50 g), HATU (1.5 eq.) and anhydrous DMF (5000 mL) were added into a single-necked reaction flask in sequence, stirred until dissolved, then added with triethylamine (3.0 eq.) slowly, and kept at room temperature for reaction. Under TLC monitoring, when the raw materials were completely reacted, 5000 mL of water was added into the reaction solution to quench the reaction, and then 5000 mL of ethyl acetate (EA) was added for extraction and layer separation. The organic phase was taken, washed three times with water (5000 mL × 3), dried with anhydrous sodium sulfate, concentrated to a certain extent (about 300 mL of EA was remained) to precipitate a large amount of solid, cooled to -5-0 °C, stirred to precipitate a solid, and then subjected to suction filtration. The filter cake was washed by dribbling a small amount of cold EA, and dried under vacuum at 45 °C for 2 h to obtain 60.8 g of the target product (90% yield). Compound I-6 was a white solid (98% purity) with a melting point of 156-158 °C. ¹H NMR (400 MHz, DMSO-*d₆*) δ 8.56 (t, *J* = 6.0 Hz, 1 H), 7.95 (d, *J* = 7.5 Hz, 2 H), 7.81 (d, *J* = 7.5 Hz, 2 H), 4.48 (t, *J* = 5.5 Hz, 1 H), 3.29 (s, 6 H), 3.29-3.25 (m, 2 H); MS (EI): m/z 336 (M + H⁺).

### Preparation Example 9

### Preparation of Compound I-7

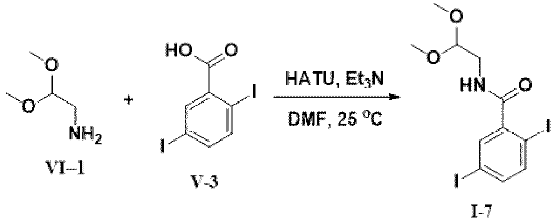

Compound I-7 was prepared by Method B. The specific reaction steps were described below.

Under nitrogen protection, Compound VI-1 (1.2 eq.), Compound V-3 (1.0 eq., 50 g), HATU (1.5 eq.) and anhydrous DMF (5000 mL) were added into a single-necked reaction flask in sequence, stirred until dissolved, then added with triethylamine (3.0 eq.) slowly, and kept at room temperature for reaction. Under TLC monitoring, when the raw materials were completely reacted, 5000 mL of water was added into the reaction solution to quench the reaction, and then 5000 mL of ethyl acetate (EA) was added for extraction and layer separation. The organic phase was taken, washed three times with water (5000 mL × 3), dried with anhydrous sodium sulfate, concentrated to a certain extent (about 300 mL of EA was remained) to precipitate a large amount of solid, cooled to -5-0 °C, stirred to precipitate a solid, and then subjected to suction filtration. The filter cake was washed by dribbling a small amount of cold EA, and dried under vacuum at 45 °C for 2 h to obtain 57.3 g of the target product (93% yield). Compound I-7 was a white solid (98% purity) with a melting point of 165-166 °C. ¹H NMR (400 MHz, DMSO-*d₆*) δ 8.57 (t, *J* = 6.0 Hz, 1 H), 8.20 (dd, *J* = 5.3*,* 1.9 Hz, 1 H), 7.90 (dd, *J =* 7.6, 5.3 Hz, 1 H), 7.42 (dd, *J* = 7.6, 1.9 Hz, 1 H), 4.48 (t, *J* = 5.5 Hz, 1 H), 3.29 (s, 6 H), 3.29-3.25 (m, 2 H); MS (EI): m/z 462 (M + H⁺).

### Preparation Example 10

### Preparation of Compound I-8

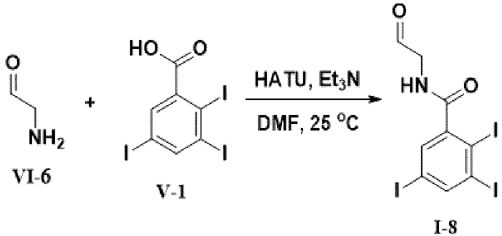

Compound I-8 was prepared by Method B. The specific reaction steps were described below.

Under nitrogen protection, Compound VI-6 (1.5 eq.), Compound V-1 (1.0 eq., 50 g), HATU (1.5 eq.) and anhydrous DMF (5000 mL) were added into a single-necked reaction flask in sequence, stirred until dissolved, then added with triethylamine (3.0 eq.) slowly, and kept at room temperature for reaction. Under TLC monitoring, when the raw materials were completely reacted, 5000 mL of water was added into the reaction solution to quench the reaction, and then 5000 mL of ethyl acetate (EA) was added for extraction and layer separation. The organic phase was taken, washed three times with water (5000 mL × 3), dried with anhydrous sodium sulfate, concentrated to a certain extent (about 300 mL of EA was remained) to precipitate a large amount of solid, cooled to -5-0 °C, stirred to precipitate a solid, and then subjected to suction filtration. The filter cake was washed by dribbling a small amount of cold EA, and dried under vacuum at 45 °C for 2 h to obtain 43.7 g of the target product (81% yield). Compound I-8 was a white solid (98% purity) with a melting point of 90-92 °C. ¹H NMR (400 MHz, DMSO-*d₆*) δ 9.25 (s, 1 H), 8.74 (t, *J =* 5.8 Hz, 1 H), 8.27 (d, *J =* 1.9 Hz, 1 H), 7.46 (d, *J* = 1.9 Hz, 1 H), 5.18 (t, *J* = 5.3 Hz, 1 H), 4.78 (d, *J =* 5.3 Hz, 2 H); MS (EI): m/z 542 (M + H⁺).

### Preparation Example 11

### Preparation of Compound I-9

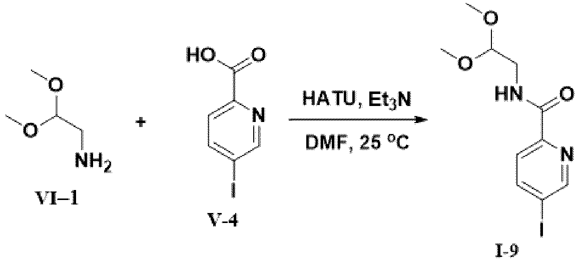

Compound I-9 was prepared by Method B. The specific reaction steps were described below.

Under nitrogen protection, Compound VI-1 (1.2 eq.), Compound V-4 (1.0 eq., 50 g), HATU (1.5 eq.) and anhydrous DMF (5000 mL) were added into a single-necked reaction flask in sequence, stirred until dissolved, then added with triethylamine (3.0 eq.) slowly, and kept at room temperature for reaction. Under TLC monitoring, when the raw materials were completely reacted, 5000 mL of water was added into the reaction solution to quench the reaction, and then 5000 mL of ethyl acetate (EA) was added for extraction and layer separation. The organic phase was taken, washed three times with water (5000 mL × 3), dried with anhydrous sodium sulfate, concentrated to a certain extent (about 300 mL of EA was remained) to precipitate a large amount of solid, cooled to -5-0 °C, stirred to precipitate a solid, and then subjected to suction filtration. The filter cake was washed by dribbling a small amount of cold EA, and dried under vacuum at 45 °C for 2 h to obtain 48.6 g of the target product (72% yield). Compound I-9 was a white solid (98% purity) with a melting point of 178-180 °C. ¹H NMR (400 MHz, DMSO-*d₆*) δ 8.56 (t, *J =* 6.0 Hz, 1 H), 9.22 (d, *J =* 1.5 Hz, 1 H), 8.76 (dd, *J* = 7.5, 1.5 Hz, 1 H), 8.13 (d, *J* = 1.5 Hz, 1 H), 4.48 (t, *J* = 5.4 Hz, 1 H), 3.29 (s, 6 H), 3.29-3.25 (m, 2 H); MS (EI): m/z 337 (M + H⁺).

### Preparation Example 12

### Preparation of Compound I-10

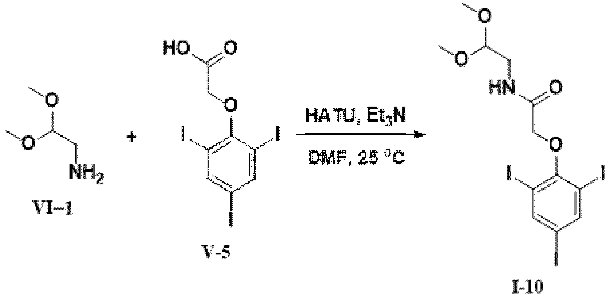

Compound I-10 was prepared by Method B. The specific reaction steps were described below.

Under nitrogen protection, Compound VI-1 (1.2 eq.), Compound V-5 (1.0 eq., 50 g), HATU (1.5 eq.) and anhydrous DMF (5000 mL) were added into a single-necked reaction flask in sequence, stirred until dissolved, then added with triethylamine (3.0 eq.) slowly, and kept at room temperature for reaction. Under TLC monitoring, when the raw materials were completely reacted, 5000 mL of water was added into the reaction solution to quench the reaction, and then 5000 mL of ethyl acetate (EA) was added for extraction and layer separation. The organic phase was taken, washed three times with water (5000 mL × 3), dried with anhydrous sodium sulfate, concentrated to a certain extent (about 300 mL of EA was remained) to precipitate a large amount of solid, cooled to -5-0 °C, stirred to precipitate a solid, and then subjected to suction filtration. The filter cake was washed by dribbling a small amount of cold EA, and dried under vacuum at 45 °C for 2 h to obtain 52.4 g of the target product (90% yield). Compound I-10 was a white solid (98% purity) with a melting point of 170-171 °C. ¹H NMR (400 MHz, DMSO-*d₆*) δ 8.55 (t, *J =* 6.0 Hz, 1 H), 7.90 (s, 2 H), 4.48 (t, *J =* 5.4 Hz, 1 H), 4.43 (s, 2 H), 3.29 (s, 6 H), 3.29-3.25 (m, 2 H); MS (EI): m/z 618 (M + H⁺).

### Preparation Example 13

### Preparation of Compound I-11

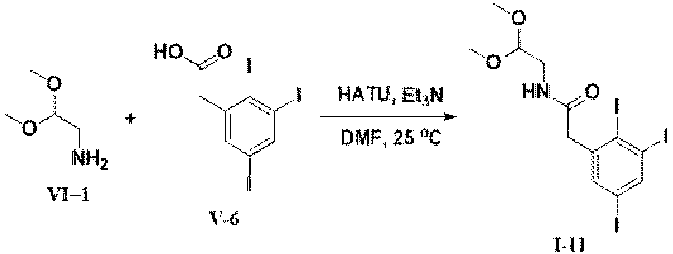

Compound I-11 was prepared by Method B. The specific reaction steps were described below.

Under nitrogen protection, Compound VI-1 (1.2 eq.), Compound V-6 (1.0 eq., 50 g), HATU (1.5 eq.) and anhydrous DMF (5000 mL) were added into a single-necked reaction flask in sequence, stirred until dissolved, then added with triethylamine (3.0 eq.) slowly, and kept at room temperature for reaction. Under TLC monitoring, when the raw materials were completely reacted, 5000 mL of water was added into the reaction solution to quench the reaction, and then 5000 mL of ethyl acetate (EA) was added for extraction and layer separation. The organic phase was taken, washed three times with water (5000 mL × 3), dried with anhydrous sodium sulfate, concentrated to a certain extent (about 300 mL of EA was remained) to precipitate a large amount of solid, cooled to -5-0 °C, stirred to precipitate a solid, and then subjected to suction filtration. The filter cake was washed by dribbling a small amount of cold EA, and dried under vacuum at 45 °C for 2 h to obtain 48.5 g of the target product (83% yield). Compound I-11 was a white solid (98% purity) with a melting point of 172-173 °C. ¹H NMR (400 MHz, DMSO-*d₆*) δ 8.50 (t, *J =* 5.7 Hz, 1 H), 7.61 (d, *J =* 2.3 Hz, 1 H), 7.47 (d, *J* = 2.3 Hz, 1 H), 4.48 (t, *J* = 5.5 Hz, 1 H), 3.85 (s, 2 H), 3.29 (s, 6 H), 3.29-3.25 (m, 2 H); MS (EI): m/z 602 (M + H⁺).

### Comparative Example 1

### Preparation of Compound I-1 via acyl chloride intermediate

1) 10 g of 2,3,5-triiodobenzoic acid and 150 g of sulfoxide chloride were added into a single-necked flask and then heated to reflux at 60 °C for 6 hours. The excess sulfoxide chloride was removed by distillation at 60 °C under reduced pressure. The remaining product was dissolved by 7 mL of dry dichloromethane with stirring, then the solution was added slowly dropwise to 100 mL of anhydrous n-hexane for precipitation, and the solid was filtered out and dried under vacuum at room temperature to obtain a tan solid, 2,3,5-triiodobenzoyl chloride (8.72 g). 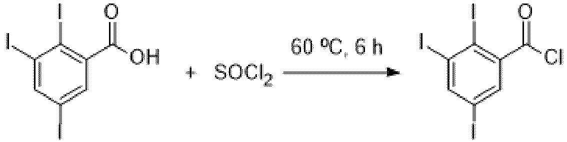
2) 1.2 g of aminoacetaldehyde dimethyl acetal was taken and dissolved in 10 mL of dimethyl sulfoxide, and then the system was added with 2 mL of 3 mol/L sodium hydroxide solution, stirred uniformly, and purged and protected with inert gas. After the system was cooled to -5 °C, 5.2 g of 2,3,5-triiodobenzoyl chloride was dissolved in 50 mL of dimethyl sulfoxide, added slowly dropwise into the reaction solution with a dropping funnel, and reacted at 30 °C for 2 h. After the reaction, the system was added with water, extracted twice with ethyl acetate, and then washed with saturated salt solution. The organic phase was dried with anhydrous sodium sulfate, filtered and subjected to rotary evaporation to obtain a light yellow solid, N-(2,2-dimethoxy ethyl)-2,3,5-triiodobenzamide.

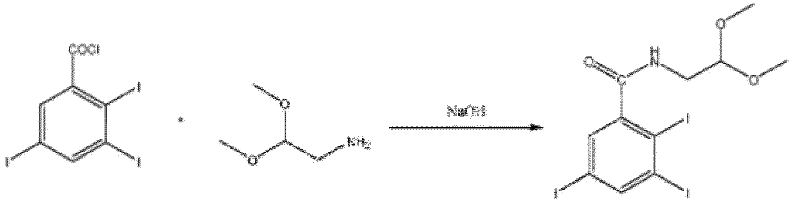

### Analysis:

Comparing the examples with Comparative Example 1 in the preparation process, the examples adopt one-step reaction to prepare N-(2,2-dimethoxyethyl)-2,3,5-triiodobenzamide or its analogs, while the comparative example requires two-step reaction, and the intermediate 2,3,5-triiodobenzoyl chloride or its analogs is unstable, easily decomposed and uneasily storable, which needs to be prepared freshly for utilization and is unfavorable to industrial production and scale-up. The remaining 2,3,5-triiodobenzoyl chloride or its analogs will also affect the subsequent reaction with embolic microspheres. In addition, Comparative Example 1 requires highly corrosive and dangerous reagents such as sulfoxide chloride and sodium hydroxide, has harsh reaction conditions, violently exothermic reaction process, lots of side reactions, high equipment requirements, and uneasy operation, and is not conducive to large-scale production.

### Comparative Example 2

The preparation of Compound I-1 in this comparative example differs from Method III of Preparation Example 1 in that the condensation reagent HATU used was replaced with an equal moles of the condensation reagent N,N'-carbonyl diimidazole (CDI), and the obtained target white solid product I-1 (5.9 g, 10% yield) has a much lower yield.

### Analysis of Acid Environment Stability

HPLC method: An Agilent C18 alkyl silica column was used, the mobile phase was 0.1% phosphoric acid/methanol, the elution was carried out at a column flow rate of 1.0 mL/mL, and the detection wavelength was 245 nm.

### Standard curve protraction

### Synthesis of the amide impurity IV-1

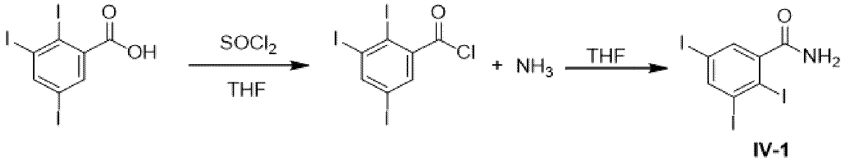

The 2,3,5-triiodobenzoic acid (10 g, 20.01 mmol, 1.0 eq) and anhydrous THF (200 mL) were added into a 500 mL single-necked reaction flask in sequence, stirred until dissolved, and then slowly added with sulfoxide chloride (11.9 g, 100.04 mmol, 5.0 eq) dropwise at a controlled temperature of 0 °C. The system was heated to 55 °C and subjected to reflux reaction for 4 h, and under TLC monitoring (a small amount of reaction solution was taken and quenched with methanol), when the raw materials were completely reacted, the reaction solution was concentrated thoroughly to dryness to obtain an off-white acyl chloride solid; meanwhile, ammonium hydroxide (30 mL, relative density ρ = 0.879 (15 °C, 28% NH₃), 26.37 g, 1.56 mol, 78 eq) was added into another 500 mL single-necked flask, then cooled to -5-0 °C, and slowly added with the acyl chloride solution prepared (the prepared acyl chloride solid was dissolved with 200 mL of anhydrous THF) dropwise. Solid was precipitated out with the dropwise addition, and after the addition, lots of solid was precipitated out. The system was stirred continuously to react for 30 min, and the reaction solution was sampled and subjected to TCL, and sent to LC-MS. When the reaction was completed, the system was subjected to suction filtration, the filter cake was washed 1-2 times by dribbling water, and the filtrate was subjected to rotary evaporation to remove THF and precipitate solid, and then subjected to suction filtration again. The solids obtained from the two suction filtrations were combined, pulped with refluxed DCM, subjected to suction filtration again, and dried to obtain 10.6 g of the target product (~100% yield). Compound IV is a white solid (98.94% purity) with a melting point of 275-277 °C. ¹H NMR (400 MHz, DMSO-*d₆*) δ 8.25 (d, *J* = 1.9 Hz, 1 H), 7.89 (s, 1 H), 7.60 (s, 1 H), 7.52 (d, *J =* 1.9 Hz, 1 H); MS (EI): m/z 500 (M + H⁺).

Compound IV-1 was prepared at different concentrations and analyzed by the above HPLC method, and the standard curve was protracted with a peak area as the horizontal coordinate and a compound concentration as the vertical coordinate.

Sample treatment: 0.5 mg of the prepared Compounds I-1 to I-11 and Compounds I-1' to I-2' were weighed out, dissolved in 10 mL of a DMSO-acid solution (9.9 mL DMSO + 0.1 mL methanesulfonic acid), and mixed and incubated with a rotary mixer for 6 h.

Analysis: 10 µL of the prepared sample after the above sample treatment was taken, and the reaction solution was diluted 100 times with DMSO and then determined by high performance liquid chromatography, and an amide impurity content was calculated by the standard curve.

The amide impurities corresponding to other compounds in the preparation examples were determined in a similar manner to the above analytical method.

### Results:

**Table 1: Compound stability test**

| Sample No. | Compound I-1 | Compound I-1' | Compound I-2 | Compound 1-2' | Compound I-3 | Compound I-4 | Compound I-5 |
|---|---|---|---|---|---|---|---|
| Impurity Content (mg/mL) | 32 | 30 | 0.61 | 0.63 | 0.69 | 0.83 | 1.49 |

| Sample No. | Compound I-6 | Compound 1-7 | Compound I-8 | Compound 1-9 | Compound 1-10 | Compound 1-11 | |
|---|---|---|---|---|---|---|---|
| Impurity Content (mg/mL) | 29.2 | 28.9 | 27.4 | 19.7 | 19.1 | 18.9 | |

Analysis: It can be found from the results in Table 1 that when n = 0, because there is only one C atom between acetal and the nitrogen atom and the acetal group is active, the compound is hydrolyzed by acid to produce Compound III and further hydrolyzed by acid to produce the amide Compound IV. With the extension of the C chain, the aldehyde Compound III tends to be stable and is not easily decomposed to produce Compound IV.

The mechanism of the hydrolysis reaction is shown as follows:

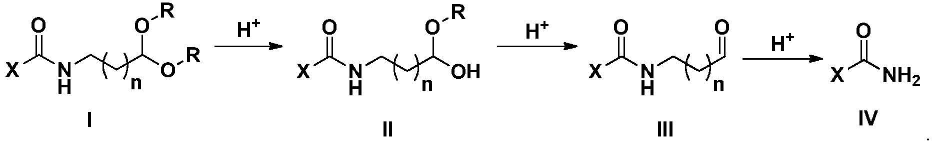

### Application Example 1

### Preparation of a radiopaque imaging microsphere

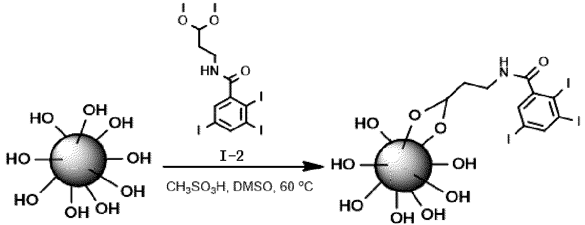

In a 250 mL three-necked round-bottom flask equipped with an overhead stirrer and a thermometer, 4.0 g of a dry polyvinyl alcohol microsphere was added and subjected to swelling with 120 mL of DMSO; 8 g of Compound I-2 and 1 mL of methanesulfonic acid were added and reacted at 60 °C for 24 h. Finally, the imaging microsphere was obtained after washed by MDSO and a sodium bicarbonate solution.

### Application Example 2

### Preparation of a radiopaque imaging microsphere

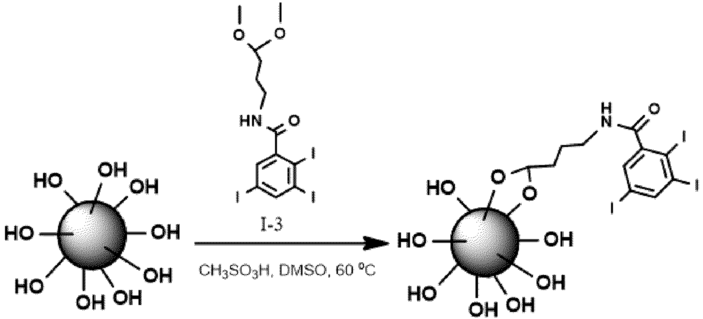

In a 250 mL three-necked round-bottom flask equipped with an overhead stirrer and a thermometer, 4.0 g of a dry polyvinyl alcohol microsphere was added and subjected to swelling with 120 mL of DMSO; 8 g of Compound I-3 and 1 mL of methanesulfonic acid were added and reacted at 60 °C for 24 h. Finally, the imaging microsphere was obtained after washed by MDSO and a sodium bicarbonate solution.

### Application Example 3

### Preparation of a radiopaque imaging microsphere

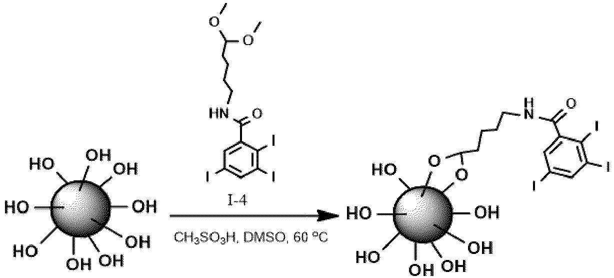

In a 250 mL three-necked round-bottom flask equipped with an overhead stirrer and a thermometer, 4.0 g of a dry polyvinyl alcohol microsphere was added and subjected to swelling with 120 mL of DMSO; 8 g of Compound I-4 and 1 mL of methanesulfonic acid were added and reacted at 60 °C for 24 h. Finally, the imaging microsphere was obtained after washed by MDSO and a sodium bicarbonate solution.

### Application Example 4

### Preparation of a radiopaque imaging microsphere

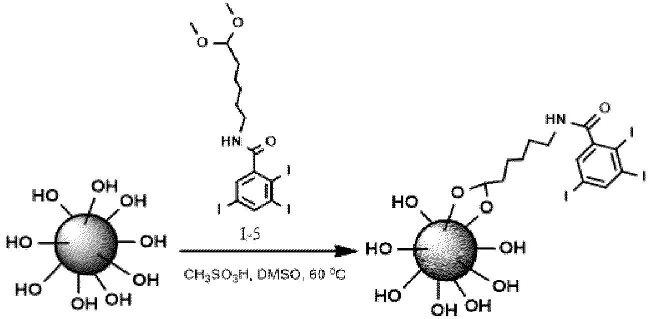

In a 250 mL three-necked round-bottom flask equipped with an overhead stirrer and a thermometer, 4.0 g of a dry polyvinyl alcohol microsphere was added and subjected to swelling with 120 mL of DMSO; 8 g of Compound I-5 and 1 mL of methanesulfonic acid were added and reacted at 60 °C for 24 h. Finally, the imaging microsphere was obtained after washed by DMSO and a sodium bicarbonate solution.

### Application Example 5

### Preparation of a radiopaque imaging microsphere

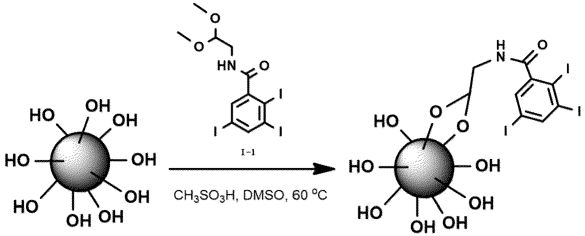

In a 250 mL three-necked round-bottom flask equipped with an overhead stirrer and a thermometer, 4.0 g of a dry polyvinyl alcohol microsphere was added and subjected to swelling with 120 mL of DMSO; 8 g of Compound I-1 and 1 mL of methanesulfonic acid were added and reacted at 60 °C for 24 h. Finally, the imaging microsphere was obtained after washed by DMSO and a sodium bicarbonate solution.

### X-ray imaging performance test of the microsphere:

The polyvinyl alcohol X-ray imageable embolic microspheres prepared by Application Examples 1-5 were soaked in a physiological saline solution and subjected to swelling equilibrium in vials. 1 mL of the imageable embolic microsphere was placed on white filter paper and observed by a medical angiography X-ray equipment (DSA, Wandong Medical, CGO-2100) under the condition of X-ray fluoroscopy. With the X-ray fluoroscopy, the radiopaque round microspheres can be clearly observed through the DSA subtraction mode, and each microsphere is clearly and completely imaged, which satisfies the X-ray visualization requirements.

The optical image results of microsphere microscopy analysis are shown in FIG. 7-a to FIG. 7-e, and the results are shown in Table 1.

**Table 1**

| Group | Figure | Result Analysis |
|---|---|---|
| Application Example 1 | FIG. 7-a | Round microsphere, good morphology, good hydrophilicity |
| Application Example 2 | FIG. 7-b | Round microsphere, good morphology, good hydrophilicity |
| Application Example 3 | FIG. 7-c | Round microsphere, good morphology, good hydrophilicity |
| Application Example 4 | FIG. 7-d | Round microsphere, good morphology, good hydrophilicity |
| Application Example 5 | FIG. 7-e | Round microsphere, rough surface, poor hydrophilicity |

### Analysis of impurities in the imaging microspheres:

### 1. Synthesis of standard substances

The 2,3,5-triiodobenzoic acid (10 g, 20.01 mmol, 1.0 eq) and anhydrous THF (200 mL) were added into a 500 mL single-necked reaction flask in sequence, stirred until dissolved, and then slowly added with sulfoxide chloride (11.9 g, 100.04 mmol, 5.0 eq) dropwise at a controlled temperature of 0 °C. The system was heated to 55 °C and subjected to reflux reaction for 4 h, and under TLC monitoring (a small amount of reaction solution was taken and quenched with methanol), when the raw materials were completely reacted, the reaction solution was concentrated thoroughly to dryness to obtain an off-white acyl chloride solid; meanwhile, ammonium hydroxide (30 mL, relative density ρ = 0.879 (15 °C, 28% NH₃), 26.37 g, 1.56 mol, 78 eq) was added into another 500 mL single-necked flask, then cooled to -5-0°C, and slowly added with the acyl chloride solution prepared (the prepared acyl chloride solid was dissolved with 200 mL of anhydrous THF) dropwise. Solid was precipitated out with the dropwise addition, and after the addition, lots of solid was precipitated out. The system was stirred continuously to react for 30 min, and the reaction solution was sampled and subjected to TCL, and sent to LC-MS. When the reaction was completed, the system was subjected to suction filtration, the filter cake was washed 1-2 times by dribbling water, and the filtrate was subjected to rotary evaporation to remove THF and precipitate solid, and then subjected to suction filtration again. The solids obtained from the two suction filtrations were combined, pulped with refluxed DCM, subjected to suction filtration again, and dried to obtain 10.6 g of the target product (~100% yield). Compound IV is a white solid (98.94% purity) with a melting point of 275-277 °C. ¹H NMR (400 MHz, DMSO-*d₆*) δ 8.25 (d, *J* = 1.9 Hz, 1 H), 7.89 (s, 1 H), 7.60 (s, 1 H), 7.52 (d, *J =* 1.9 Hz, 1 H); MS (EI): m/z 500 (M + H⁺).

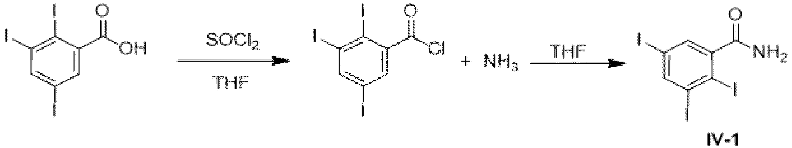

### 2. HPLC standard curve protraction

Different amounts of the above prepared standard substance were taken and diluted 100 times with DMSO and then determined by high performance liquid chromatography; test method: an Agilent C18 alkyl silica column was used, the mobile phase was 0.1% phosphoric acid and methanol, the elution was carried out at a column flow rate of 1.0 mL/mL, and the wavelength was 245 nm; a peak area of the standard substance was obtained and formed a standard curve as shown in FIG. 9.

### 3. Impurity content determination in Application Examples 1-5

After the imaging modification reaction of the microsphere was completed, with reference to the above HPLC method, 10 µL of the reaction solution was taken, diluted 100 times with DMSO and determined by HPLC. The results are shown in FIG. 8-a to FIG. 8-e, and the data are summarized in Table 2.

4. Strength and elasticity are important indicators to characterize the embolic performance of embolic microspheres. Generally, the strength of embolic microspheres should not be higher than 500 g force, and should return to spherical shape after compression. The microspheres were laid flat onto a glass slide and arranged in a monolayer, and the strength and elasticity were determined by a texture analyser (TA-XTplusC). The strength was defined as the maximum force during 50% compression, and the elasticity was evaluated by whether the microspheres could return to spherical shape after 50% compression, and the results are shown in Table 2.

**Table 2**

| Sample No. | Figure | Impurity Content of Reaction Solution (mg/mL) | Iodine Content of Dry Microsphere | Iodine Content of Wet Microsphere (mg/mL) | Strength | Elasticity |
|---|---|---|---|---|---|---|
| Application Example 1 | FIG. 8-a | 0.61 | 47% | 178 | 431.15 g | Yes |
| Application Example 2 | FIG. 8-b | 0.52 | 46% | 172 | 375.23 g | Yes |
| Application Example 3 | FIG. 8-c | 0.50 | 43% | 166 | 257.12 g | Yes |
| Application Example 4 | FIG. 8-d | 0.55 | 30% | 102 | 143.14 g | Yes |
| Application Example 5 | FIG. 8-e | 26 | 27% | 97 | 2031.15 g | No |

By comparing the results, it can be seen that the amide impurity IV generated during the reaction is significantly reduced by increasing the carbon chain length in the examples, and the difficulty of cleaning is reduced. Because side reactions are reduced, more imaging molecules are bonded to microspheres, thus significantly increasing the iodine content of the imaging microspheres. The carbon chain of Example 4 is too long (n ≥ 4), and the imaging molecule is too large, the imaging modification efficiency may turn out to be reduced, resulting in a decrease in the iodine content of dry microsphere and wet microsphere. In terms of mechanical properties, the strength in Application Example 5 is as high as 2031 g, which is much higher than the requirement of 500 g, and the microsphere cannot return to spherical shape after compression, and the embolic performance will be poor.

### Drug delivery performance test

1 mL of the microspheres in Application Examples 1-5 were measured separately, the upper preservation solution was removed, 2 mL of a solution containing 40 mg of adriamycin hydrochloride was added, and the system was gently shaken, sampled at 30 min and 24 h, respectively, and determined by HPLC for the content of adriamycin. The results are shown in Table 3.

**Table 3**

| Sample No. | Drug Load Capacity (24 h) | Drug Delivery Rate |
|---|---|---|
| Application Example 1 | 35 mg/mL | 30 mg/30 min |
| Application Example 2 | 40 mg/mL | 35 mg/30 min |
| Application Example 3 | 41 mg/mL | 40 mg/30 min |
| Application Example 4 | 43 mg/mL | 38 mg/30 min |
| Application Example 5 | 20 mg/mL | 15 mg/30 min |

It can be seen that the compound of the present application, when used in the imaging microsphere, can bring the imaging microsphere a higher drug delivery rate and a larger drug loading capacity.

Applicant declares that although the amide compound having iodoaryl or iodoheteroaryl, the preparation method therefor and use thereof of the present application are illustrated by the embodiments in the present application, the present application is not limited to the embodiments, which means that the present application is not necessarily rely on the embodiments to be implemented. It should be apparent to those skilled in the art that any improvement of the present application, equivalent substitution of the raw materials selected for the present application, the addition of auxiliary ingredients, the specific methods selected, etc., all fall within the protection scope and disclosure scope of the present application.

## Claims

1. An amide compound having iodoaryl or iodoheteroaryl, which has a structure shown in formula I: 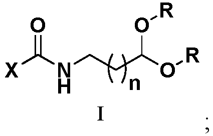 wherein X is C₅₋₁₂ aryl substituted by at least one iodine atom, C₅₋₁₂ heteroaryl substituted by at least one iodine atom, C₅₋₁₂ aryloxy-C₁₋₄ alkylene substituted by at least one iodine atom, or C₅₋₁₂ aryl-C₁₋₄ alkylene substituted by at least one iodine atom; R is C₁₋₆ alkyl; and n is an integer greater than or equal to 0.

2. The amide compound having iodoaryl or iodoheteroaryl according to claim 1, wherein for the compound of formula I, n is equal to 0, 1, 2 or 3.

3. The amide compound having iodoaryl or iodoheteroaryl according to claim 1, wherein X is C₅₋₇ aryl substituted by at least one iodine atom.

4. The amide compound having iodoaryl or iodoheteroaryl according to claim 1, wherein X is phenyl substituted by at least one iodine atom.

5. The amide compound having iodoaryl or iodoheteroaryl according to claim 1, wherein X is selected from any one of the following groups: 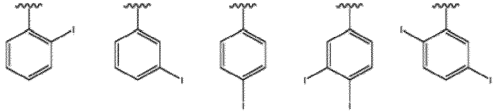 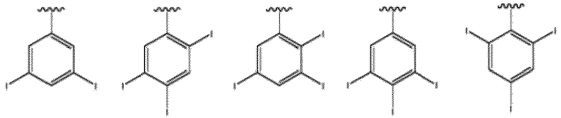 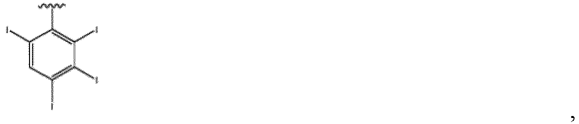
wherein the wavy line represents a linkage site of a group;
preferably, R is methyl, ethyl, n-propyl, isopropyl, n-butyl or n-hexyl;
preferably, the amide compound having iodoaryl or iodoheteroaryl is any one of the following compounds:

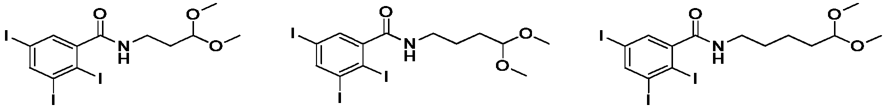

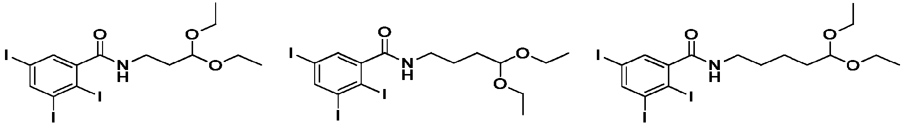

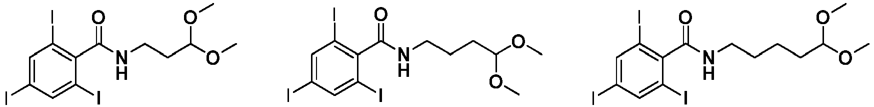

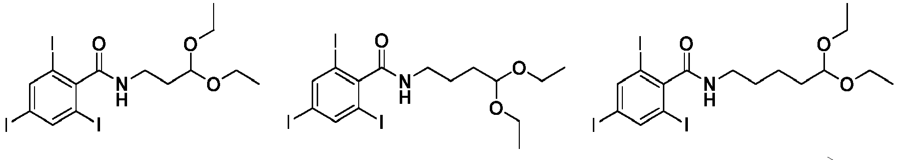

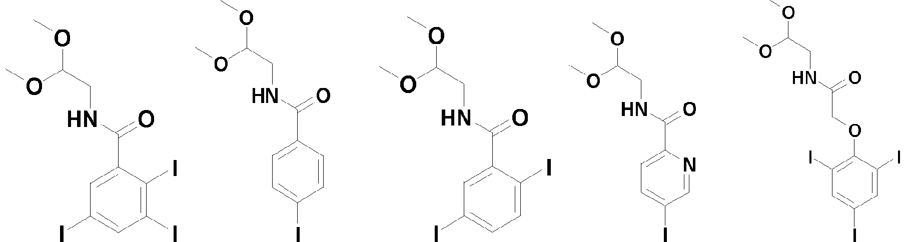

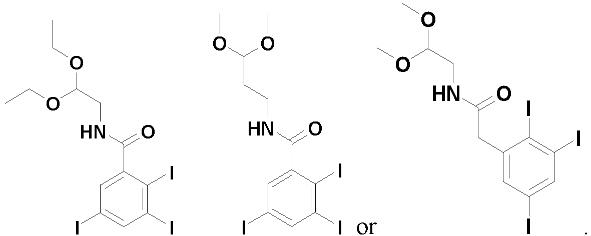

6. A method of preparing the amide compound having iodoaryl or iodoheteroaryl according to any one of claims 1 to 5, comprising:
carrying out a condensation reaction on a compound of formula V and a compound of formula VI to obtain the amide compound having iodoaryl or iodoheteroaryl shown in formula I, the reaction formula of which is as follows: 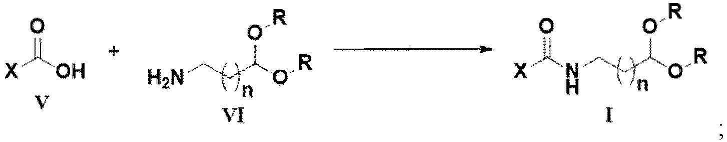
wherein X, R and n are defined as in claim 1;
preferably, the compound of formula VI and the compound of formula V have a molar ratio of 1:1-10:1;
preferably, the condensation reaction is carried out in the presence of a condensation reagent;
preferably, the condensation reagent is a combination of 1-(3-dimethylaminopropyl)-3-ethylcarbodiimide hydrochloride and 1-hydroxybenzotriazole, or 2-(7-azobenzotriazo1-yl)-N,N,N',N'-tetramethyluronium hexafluorophosphate;
preferably, the condensation reagent and the compound of formula VI have a molar ratio of 1:1-6:1;
wherein the condensation reagent is a combination of 1-(3-dimethylaminopropyl)-3-ethylcarbodiimide hydrochloride and 1-hydroxybenzotriazole, 1-(3-dimethylaminopropyl)-3-ethylcarbodiimide hydrochloride and 1-hydroxybenzotriazole have a molar ratio of 1:1;
preferably, the condensation reaction is carried out in the presence of a basic substance, and the basic substance is triethylamine, N,N-diisopropylethylamine, triethylenediamine or N-methylmorpholine;
preferably, a solvent of the condensation reaction is any one or a combination of at least two of N,N-dimethylformamide, N,N-dimethylacetamide, dichloromethane or tetrahydrofuran;
preferably, the condensation reaction is carried out at room temperature for 2-48 h;
preferably, the condensation reaction is carried out under the protection of a protective gas, and the protective gas is preferably nitrogen.

7. An X-ray imaging material, comprising the amide compound having iodoaryl or iodoheteroaryl according to any one of claims 1 to 5 as an imaging molecule;
preferably, the X-ray imaging material is an X-ray imaging embolic microsphere.

8. The X-ray imaging material according to claim 7, wherein the radiopaque embolic microsphere comprises a polyvinyl alcohol microsphere and the imaging molecule which are connected in a manner of 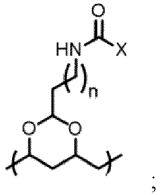
wherein X is C₅₋₁₂ aryl substituted by at least one iodine atom or C₅₋₁₂ heteroaryl substituted by at least one iodine atom; n is 0, 1, 2 or 3;
preferably, polyvinyl alcohol of the polyvinyl alcohol microsphere has a molecular mass of 2000-10000000;
preferably, the radiopaque embolic microsphere has the following structure: 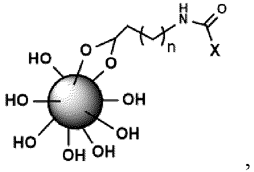 wherein 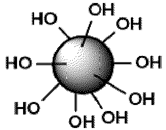 represents the polyvinyl alcohol microsphere, and the structural formula connected to the polyvinyl alcohol microsphere is derived from the imaging molecule;
preferably, the polyvinyl alcohol microsphere has an average diameter of 10-2000 µm.

9. A method of preparing the X-ray imaging material according to claim 7 or 8, comprising:
adding a polymer microsphere into a solvent for swelling, then adding the imaging molecule and methanesulfonic acid, and carrying out a reaction to obtain the X-ray imaging material;
preferably, the solvent is dimethyl sulfoxide;
preferably, in the reaction system, the polymer microsphere has a mass percent of 1%-50%;
preferably, in the reaction system, the imaging molecule has a molar concentration of 0.01-5 mol/L;
preferably, in the reaction system, the methanesulfonic acid has a molar concentration of 0.05-5 mol/L;
preferably, the reaction is carried out at 50-90 °C for 24-48 h.

10. A composition, comprising a solvent and the X-ray imaging material according to any one of claims 7 to 9;
preferably, the solvent is a physiological saline solution.
